# EUROPEAN PATENT APPLICATION

(11) **EP 2 420 503 A1**
(43) Date of publication of application: **22.02.2012**
(21) Application number: 10450123.4
(22) Date of filing: 27.07.2010
(51) Int. Cl.: C07D 489/02, C07D 489/08, A61K 31/485, A61P 25/04

(54) **Process for the synthesis of 14-oxygenated morphinan-6-ones**

(71) Applicant: Universität Innsbruck, 6020 Innsbruck (AT)
(72) Inventor: Schmidhammer, Helmut Prof.Dr.,, 6020 Innsbruck (AT)
(74) Representative: Schwarz & Partner

(57) **Abstract**

A process for the production of 14-oxygenated morphinan-6-ones wherein a methylether group in position 3 of the phenyl ring which has to be cleaved to obtain the hydroxy group, and wherein the protection of the hydroxy group in position 3 of the phenyl ring in the presence of a ketal group is avoided.

## Description

The present invention relates to the synthesis of pharmaceutically active compounds, such as 14-oxygenated morphinan-6-ones.

In WO 2004/005294 there are disclosed 14-oxygenated morphinan-6-ones which are described to be highly-active analgesics and also opoid antagonists and their production starting either from thebaine of formula 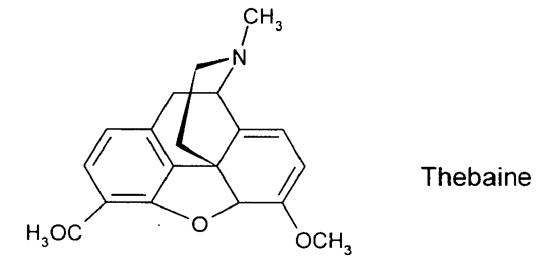 or from a cyclic ketal of formula 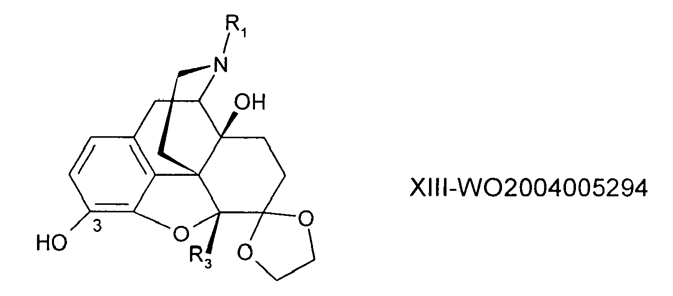 wherein the residues have various meanings.

Thebaine, however, comprises a methylether function which must be cleaved in the course of the production process to obtain a hydroxy group which generally needs rather harsh conditions - e.g. ether cleavage of the 3-O-methyl-ether is carried out with 48% HBr under reflux - and in a cyclic ketal of formula XIII in W02004/005294 the hydroxy group in position 3 of the phenyl ring is to be protected in the presence of the ketal group.

Surprisingly, now production processes for the production of 14-oxygenated morphinan-6-ones have been found wherein the protection of the hydroxy group in position 3 of the phenyl ring in the presence of a ketal group is avoided and wherein a methylether group which has to be cleaved to obtain a hydroxy group is avoided.

In one aspect the present invention provides a process for the production of an 14-oxygenated morphinan-6-one, wherein a methylether group in position 3 of the phenyl ring which has to be cleaved to obtain the hydroxy group, and wherein the protection of the hydroxy group in position 3 of the phenyl ring in the presence of a ketal group is avoided.

A process for the production of 14-oxygenated morphinan-6-ones which is provided according to the present invention is herein also designated as "Process(es) of (according to) the present invention".

An 14-oxygenated morphinan-6-one in a process according to the present invention includes a compound of formula 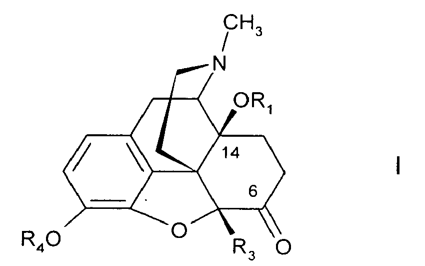 wherein
R₁ is alkyl, alkenyl, alkynyl, cycloalkylalkyl, aralkyl, alkanoyl, alkenoyl, alkynoyl, cycloalkylcarbonyl, aroyl, or alkynoyl substituted by cycloalkyl,
R₃ is alkyl, alkenyl, alkynyl, cycloalkylalkyl or aralkyl, or alkynoyl substituted by cycloalkyl, and
R₄ is hydrogen or has the meaning of R₁.

In one aspect R₁ is alkyl, alkenyl, alkynyl, cycloalkylalkyl, aralkyl, alkanoyl, alkenoyl, alkynoyl, cycloalkylcarbonyl, aroyl. In another aspect R₁ is alkyl, alkenyl, alkynyl, alkanoyl, alkenoyl, alkynoyl substituted by cycloalkyl, arylalkyl.

Preferably R₁ is alkyl, or aralkyl, such as methyl, butyl, benzyl.

Preferably R₃ is hydrogen.

Preferably R₄ is hydrogen.

In another aspect in a compound of formula I R₁ is alkyl, or aralkyl, R₃ is hydrogen and R₄ is hydrogen.

### Preferably

- Alkyl as defined herein includes C₁-₆alkyl, which alkyl optionally is substituted by one or more, such as 1 to 3 hydroxy, e.g. including C₁₋₆-monohydroxyalkyl; C₂₋₆-dihydroxyalkyl; C₃₋₆-trihydroxyalkyl;
- Alkenyl as defined herein includes C₂₋₆-alkenyl.
- Alkynyl as defined herein includes C₂₋₆-alkynyl.
- Cycloalkylalkyl as defined herein includes C₄₋₁₆cycloalkytalkyl, wehrein cylcloalkyl preferably is C₃₋₁₀cycloalkyl and alkyl preferably is C₁₋₆-alkyl.
- Aryl as defined herein includes C₆₋₁₂ aryl, such as C₆₋₁₀ aryl, e.g. phenyl, e.g. phenyl substituted by C₁₋₄alkyl.
- Aralkyl as defined herein includes C₇₋₁₆aralkyl, e.g. C₁₋₄alkylC₆₋₁₂aryl e.g. benzyl.
- Alkanoyl as defined herein includes C₂₋₆-alkanoyl.
- Alkenoyl as defined herein includes C₂₋₆-alkenoyl.
- Alkynoyl as defined herein includes C₂₋₆-alkynoyl.
- Alkyl, alkenyl alkynyl, alkanoyl, alkenoyl or alkynoyl may be straight chained or branched, optionally substituted by cycloalkyl, arylalkyl.
- Cycloalkylcarbonyl as defined herein includes C₃₋₁₀cycloalkylcarbonyl;
- Aroyl as defined herein includes C₆₋₁₂ aroyl, such as C₆₋₁₀ aroyl, e.g. phenoyl.
- An organic per-acid as defined herein, e.g. includes performic acid, m-chloroperbenzoic acid.

Aryl or cyloalkylalkyl as defined herein include unsubstituted or substituted aryl or cyloalkyl, e.g. mono-, di-or trisubstituted, wherein the substituents include independently of each other hydroxy, halogen, nitro, cyano, thiocyanato, trifluormethyl, C₁₋₃alkyl, C₁₋₃alkoxy, CO₂H CONH₂, COOC₁₋₃alkyl, CONH(C₁₋₃alkyl), CON(C₁₋₃alkyl)₂, CO(C₁₋₃alkyl); amino; C₁₋₃alkylamino, C₁₋₃di-alkylamino, C₅₋₆cycloalkylamino; (_{C1-3}alkanoyl)amido, SH, SO₃H, SO₃(C₁₋₃alkyl), SO₂(C₁₋₃alkyl), SO(C₁₋₃alkyl), C C₁₋₃alkylthio or C₁₋₃alkanoylthio.

In another aspect the present invention provides a process for the production of a compound of formula I, wherein R₁, R₃ and R₄ are as defined above, comprising
A1) deprotecting the protected hydroxy group in position 3 of the phenyl ring in a compound of formula 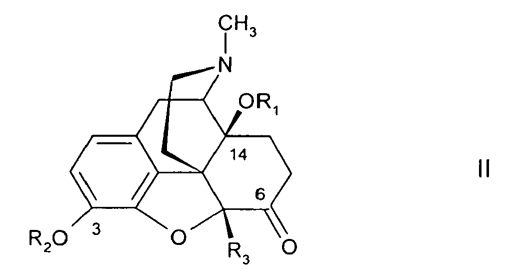 wherein R₁ and R₃ are as defined above and R₂ is a hydroyxy protecting group, or
A2) deprotecting the protected hydroxy group in position 3 of the phenyl ring and hydrolizing the enol ether in position 6 in a compound of formula 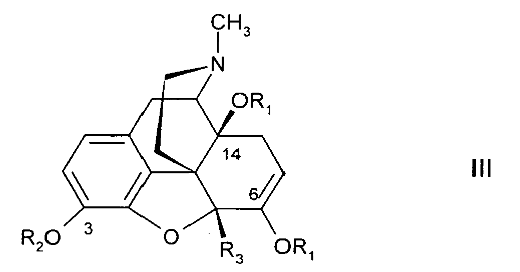 wherein R₁, and R₂ and R₃ are as defined above,
   and isolating a compound of formula I wherein R₁ is as defined above and R₄ is hydrogen from the reaction mixture, and, optionally
B) etherifying or esterifying the hydroxy group in position 3 of the phenyl ring in a compound of formula III to form a group OR₄, wherein R₄ has the meaning of R₁ as defined above.

Esterifying or etherifying includes alkylating, alkenylating, alkynylating, cycloalkylalkylating, aralkylating, alkanoylating, alkenoylating, alkynoylating, cycloalkylcarbonylating or aroylating and, esterifying or etherifying as referred to herein may be carried out as appropriate, e.g. according, e.g. analogously, to a process as conventional, or as described herein.

In one aspect of the present invention step B) is not carried out in a process provided by the present invention.

R₂ is a hydroxy protecting group, e.g. including
- a group -COR_{A} wherein R_{A} is alkyl, e.g. tert-butyl, alkyl substituted by aryl, e.g. benzyl, e.g. including methoxybenzyl, 3,4-dimethoxybenzyl,
- alkoxyalkyl, such as C₁₋₄alkoxyalkyl, e.g. methoxyalkyl including methoxymethyl (MOM), wherein alkoxy optionally is substituted by aryl, such as phenyl, e.g. including benzyloxymethyl (BOM),
- a bulky alkyl group, such as tert-butyl, cyclopropylmethyl, trityl,
- an alkenyl group, such as allyl,
- a silyl group, such as a trialkylsilyl group, e.g. including t-butyldimethylsilyl, triisopropylsilyl, trimethylsilyl.

A methyl group is not regarded as a hydroxy protecting group herein.

Protection of the hydroxy group in position 3 of the phenyl ring may be carried out as appropriate. Protection of a hydroxy group is a known reaction and may be carried out according, e.g. analogously to a method as conventional, e.g. by use of a group R₂-X, wherein X is a leaving group, such as halogen, hydroxy, in organic solvent.

In a process provided by the present invention the deprotection of the hydroxy group in position 3 of the phenyl ring in a compound of formula II, III or IV, and optionally at the same time hydrolysis of the enol ether in a compound of formula III; may be performed using rather mild hydrolytic conditions, e.g. hydrochloric acid in MeOH. For deprotection / hydrolysis either a compound of formula II or a compound of formula III, or a mixture of compounds of formula II, and/or III may be used to obtain a compound of formula I, wherein R₃ and R₄ are hydrogen and R₁ is as defined above.

Compounds of formula II, III or IV, respectively, may be obtained as appropriate, e.g. according, e.g. analogously to a method as conventional.

Preferably a compound of formula II is obtained by esterifying or etherifying the hydroxy group in position 14 in a compound of formula 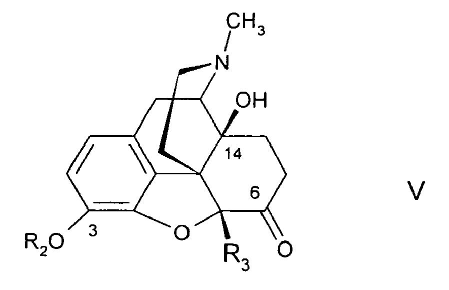 wherein R₂ and R₃ are as defined above.

Preferably a compound of formula III is obtained by esterifying or etherifying the hydroxy group in position 14 in a compound of formula V wherein R₂ and R₃ are as defined above.

Preferably a compound of formula 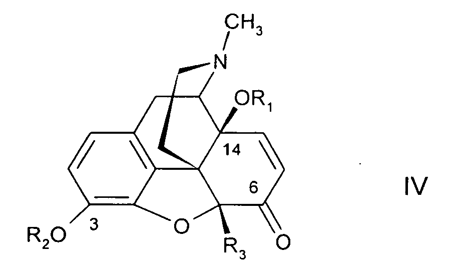 is obtained by esterifying or etherifying the hydroxy group in position 14 in a compound of formula 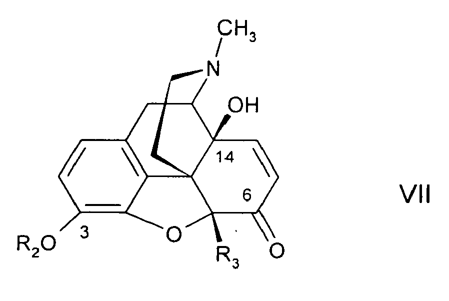 respectively, wherein R₂ and R₃ are as defined above.

R₃ is preferably H (e.g. as in 14-hydroxymorphinone), methyl or benzyl.

In another aspect the present invention provides a process according to the present invention wherein
in step A1) a compound of formula II is obtained from a compound of formula 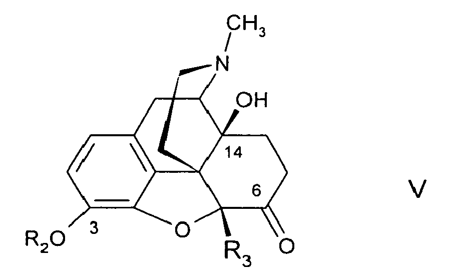 wherein R₂ and R₃ are as defined above;
in step A2) a compound of formula III is obtained from a compound of formula V, wherein R₂ and R₃ are as defined above.

A compound of formula VII may be obtained from 14-hydroxymorphinone by acylation or alkylation of the hydroxy group in position 3 of the phenyl ring.

A compound of formula II may also be obtained from a compound of formula IV, wherein R₁ and R₂ are as defined above, as appropriate, e.g. via selective hydrogenation of the double bond without cleaving off R₂, e.g. analogously to a method as conventional, or as described herein.

A compound of formula V or VII may be etherified or esterified at the hydroxy group in position 14 by reaction with an appropriate reactant to obtain a compound of formula II, III or IV, respectively, wherein R₁ is as defined above,
e.g. by reaction with a compound of formula R₁Y or (R₁)₂Y wherein R₁ is as defined above and Y is a leaving group,
e.g. Y is
- a sulfate, e.g. in a dialkylsulfate,
- a fluorosulfonic acid, e.g. in a fluorosulfonic acid ester, wherein the ester is an alkyl, alkenyl, alkynyl, cyclohexyl, aryl,
- halogen, e.g. a compound of formula R₁-Hal, wherein R₁ is as defined for a compound of formula I and Hal is halogen, or
- hydroxy, halogen or an active ester group in a compound of formula R₁-COOH, wherein the acid function is in a reactive.form, e.g. in the form of a halogenide, or in the form of an active ester.

The etherification or esterification reaction may be carried out as appropriate, e.g. according,
e.g. analogously to a method as conventional,
e.g. in organic solvent, such as *N,N-*dimethylformamide, tetrahydrofurane, 1,2-dimethoxyethane, diethylether or similar solvents in the presence of a strong base such as sodium hydride, sodium amide, potassium hydride, n-butyllithium, tert-butyllithium, lithium diethylamide, lithium diisopropylamide or similar strong bases to obtain a compound of formula II and optionally of formula III wherein R₁ is as defined above;
e.g. analogously to H.Schmidhammer et al. J. Med. Chem. 33 (1990) 1200-1206; H. Schmidhammer in Current Topics in Med. Chem. 1 (1993) 261-275; F. Schüllner et al. Helv. Chim Acta 86 (2003), 2335-2341). It is possible that during the course of the reaction morphinan-6-ones of formula III are also formed. Thus, either a compound of formula II or III, or a mixture of compounds of formula II and III are obtained. Either a compound of formula II, or a compound of formula III, or a mixture of compounds of formula II and III may be used for the subsequent deprotection / hydrolysis step to obtain a compound of formula I.

A compound of formula V may be e.g. obtained from a compound of formula 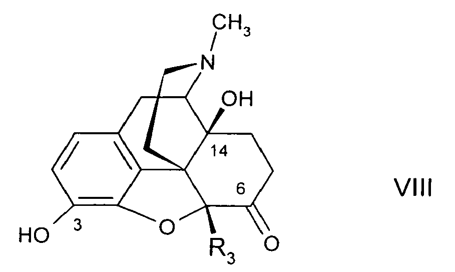 wherein R₃ is as defined above, and
a compound of formula VII may be e.g. obtained from a compound of formula 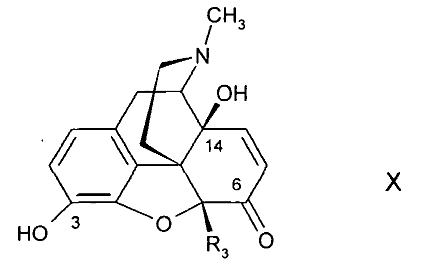 wherein R₃ is as defined above,
by protecting the hydroxy group in position 3 of the phenyl ring, e.g. by reaction with a halogenide, such as an aralkylhalogenide or a trialkylsilylhalogenide in organic solvent, e,g, polar solvent such as DMF, in the presence of a base.

A compound of formula VIII, wherein R₃ is hydrogen is oxymorphone.

A compound of formula X, wherein R₃ is hydrogen is 14-hydroxymorphinone (1).

Oripavine of formula 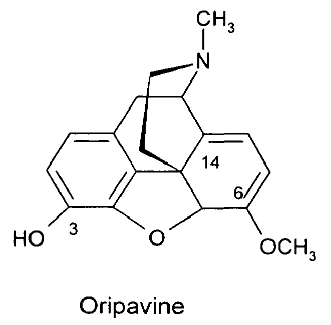 and 14-Hydroxymorphinone (1)of formula 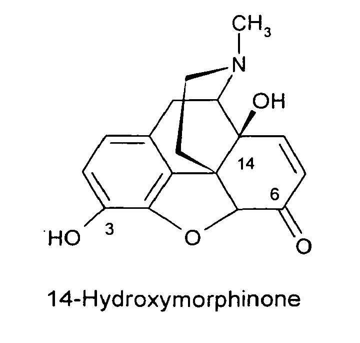 are known compounds and may be provided e.g. commercially.

Oxymorphone of formula 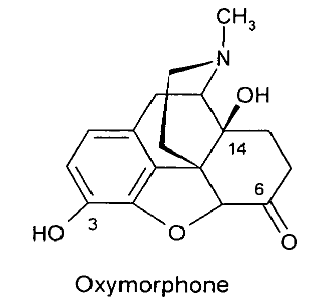 is a known compound and may be provided as appropriate, e.g. commercially.

E.g. oxymorphone may be obtained from orapivine by treatment with an organic per-acid, such as performic acid, m-chloroperbenzoic acid and subjecting the 14-hydroxymorphinone obtained to catalytic hydrogenation, e.g. over a catalyst such as Pd/C, see e.g. H. Schmidhammer et al. Helv. Chim. Acta 73 (1990), 1779-1783; or catalytic transfer hydrogenation using for instance isopropanol, cyclohexane, formic acid or ammonium formate and a catalyst, e.g. according to B.C. Ranu et al. J. Ind. Chem. Soc. 75 (1998), 690-694; S. Ram and L.D. Spicer, Synth. Commun. 22 (1992), 2683; J.B. Arterburn et al. Tetrahedron Lett. 41 (2000), 7847-7849), to obtain oxymorphone.

Starting from 14-oxygenated morphinan-6-ones of formula II obtained according to the present invention it is possible to prepare a vast number of different morphinan-6-ones such as: 6-amino acid substituted compounds and derivatives thereof, see e.g. J. Schütz et al. Helv. Chim. Acta 86 (2003), 2142-2148); DE 10161963; EP 05019796); 6-cyano compounds, see e.g. J. Schütz et al. J. Org. Chem. 70 (2005), 5323-5326; E. Greiner et al. J. Am. Chem. Soc. 123 (2001), 3840-3841) and derivatives thereof; 4,5-ring open compounds, see e.g. H. Schmidhammer et al. J. Med. Chem. 27 (1984), 1575-1579; H. Schmidhammer et al. J. Med. Chem. 32 (1989), 418-421; H. Schmidhammer et al. J. Med. Chem. 33 (1990), 1200-1206); H. Schmidhammer et al. 38 (1995), 3071-3077; M. Spetea et al. J. Med. Chem. 47 (2004), 3242-3247) and derivatives thereof; and many other in position 6 substituted 14-oxygenated morphinan-6-ones.

Any compound described herein may be obtained as appropriate, e.g. according, e.g. analogously to a method as conventional: or as described herein.

In a preferred aspect an oxymorphinone derivative of formula 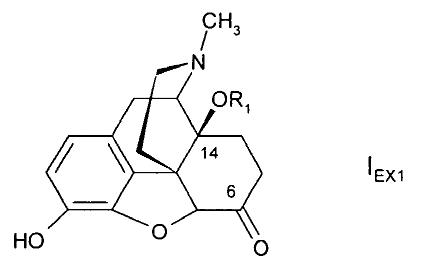 wherein R₁ is as defined above is prepared from a compound of formula 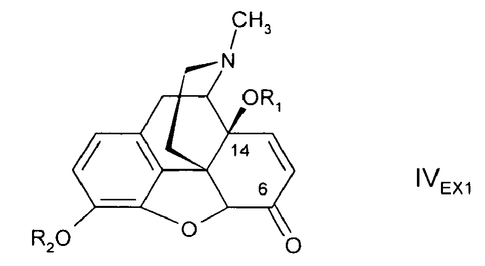 or
a compound of formula 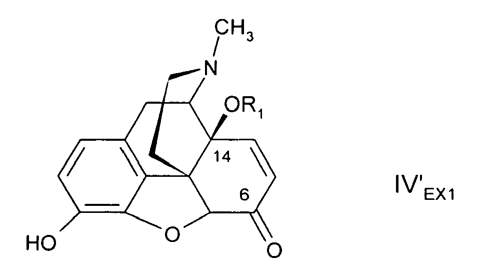 wherein R₁ and R₂ are as defined above, by catalytic transfer hydrogenation of the double bond and optionally splitting off the protection group from the hydroxy group attached to the phenyl ring preferably in the presence of an acid, e.g. formic acid, and in the presence of a catalyst, e.g. a Pd/C-catalyst;
e.g. wherein a compound of formula IV_{EXI} is obtained from a compound of formula 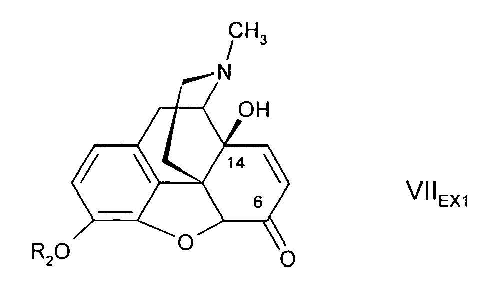 wherein R₂ is as defined above e.g. by reaction with a compound of formula L-R₁, wherein L is a leaving group;
e.g. wherein a compound of formula IV'_{EXI} is obtained from a compound of formula IV_{EXI} by splitting off the hydroxy protecting group from the hydroxy group attached to the phenyl ring in position 3;
e.g. wherein a compound of formula VII_{EXI} is obtained from 14-hydroxymorphinone (1) by protection of the hydroxy group in position 3 of the phenyl ring.

In another preferred aspect an oxymorphone derivative of formula I_{EXI} is obtained from a compound of formula 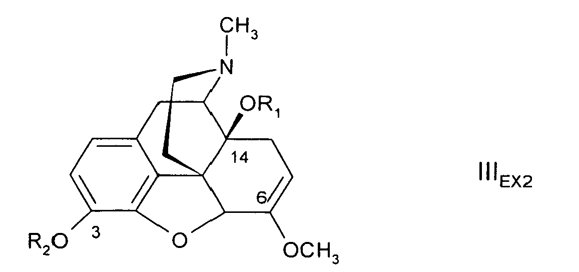 wherein R₁ and R₂ are as defined above, by acidic hydrolysis;
e.g. wherein a compound of formula III_{EX2} is obtained from a compound of formula 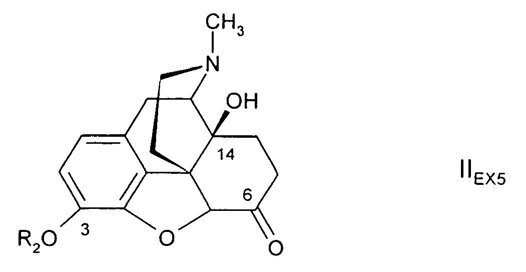 wherein R₂ is as defined above by reaction of the hydroxy group in position 14 with a compound of formula R₁-L, wherein R₁ is as defined above and L is a leaving group,
e.g. wherein a compound of formula II_{EX5} is obtained from oxymorphine by protection of the hydroxy group in position 3 of the phenyl ring.

In the following examples all temperatures are in degree C (°C) and are uncorrected.

The following abbreviations are used herein:
- abs: absolute
- conc.: concentrated
- DMF: N,N-dimethylformamide
- EtOH: ethanol
- h: hour(s)
- Mp: melting point
- MeOH: methanol
- min: minutes
- r.t.: room temperature

### Example 1

### 14-O-Methyloxymorphone from 14-Hydroxymorphinone

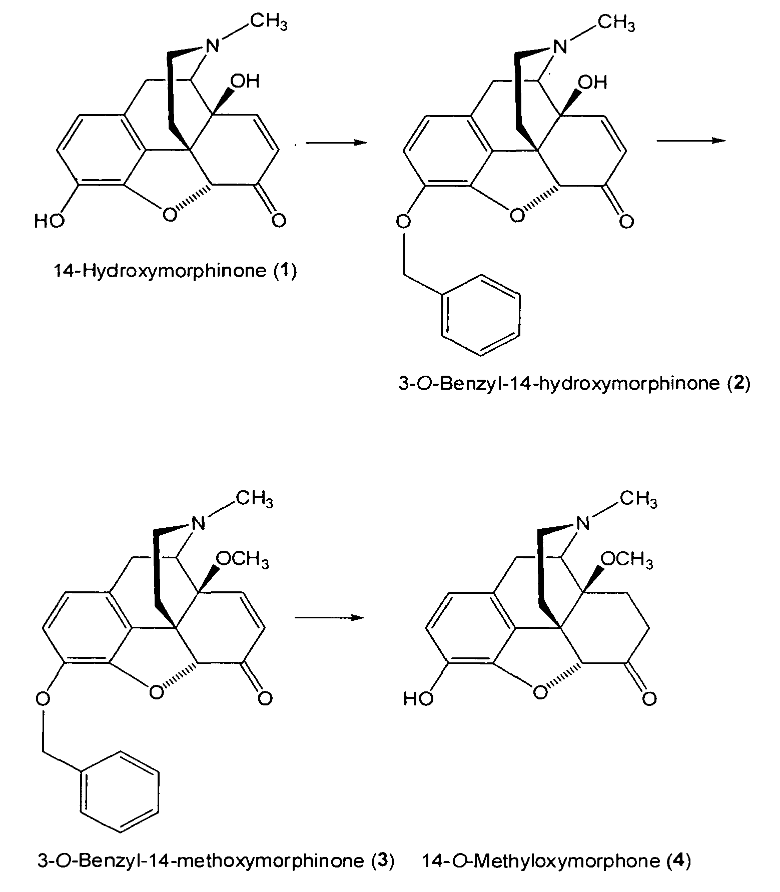

### 3-O-Benzyl-14-hydroxymorphinone (2)

A mixture of 2.00 g (6.7 mmol) of **1**, 2.80 g (20.0 mmol) K₂CO₃, 40 ml anhydrous DMF and 0.9 ml (7.4 mmol; 1.26 g) benzyl bromide was stirred under argon for 18 h at RT. After addition of 250 ml H₂O the mixture was extracted with CH₂Cl₂ (3x50 ml) and the combined organic layers were washed with H₂O (4x50 ml), dried over Na₂SO₄ and evaporated. The crystalline solid (3.80 g) was treated with 8 ml of hot MeOH, the mixture cooled and 2 was isolated as colorless crystals (Seki, I. 14-Hydroxymorphinone Derivates. Jpn. Tokkyo Koho JP 46009704, 1971; Chem. Abstr. 1971, 75, 36438).

Mp.: 246°C (mp. I Seki, s. above, 244-245°C)

IR (KBr): 3303 (OH) cm⁻¹
1673 (CO) cm⁻¹

1H-NMR (200 MHz, CDCl₃): δ [ppm] 7,42-7,26 (m, 5 arom. H)
6,72 (d, 1 arom. H, J= 8,1 Hz)
6,62 (d, 1 olef. H, J= 10 Hz)
6,57 (d, 1 arom. H, J= 8,1 Hz)
6,19 (d, 1 olef. H,J= 10 Hz)
5,16 (s, 2 H, CH₂-Phenyl)
4,73 (s, 1 H, H5)
2,44 (s, 3 H, NCH₃)

### 3-O-Benzyl-14-methoxymorohinone (3)

0.09 g of NaH (obtained by several washings of 0.26 g of a 60% NaH-dispersion in oil with petrolether) were added to a suspension from 7 mL DMF_{abs} and 1.0 g (2.6 mmol) of -*O-*Benzyl-14-hydroxymorphonone (**2**) under argon and cooling in an ice bath and the mixture obtained was stirred for 30 min under ice-cooling. To the mixture obtained 0.36 mL (3.8 mmol) of dimethyl sulfate were added and the mixture obtained was stirred for 2 h under ice-cooling. In order to complete the reaction, further 0.02 g of NaH (obtained by several washings of 0.05 g of a 60% NaH-dispersion with petrolether) were added and stirring was continued under ice-cooling for 1 h. To the mixture obtained ice was added carefully in order to stop the reaction and the mixture obtained was poured onto 45 mL of an ice-H₂O mixture. The mixture obtained was extracted with CH₂Cl₂ (4x6 mL) and the combined organic phases obtained were washed with H₂O (4x 10 mL) and dried over Na₂SO₄. From the mixture obtained solvent was evaporated and a brown viscous mixture (0.85 g) was obtained from which yellow-brownish crystalls precipitated which were treated with boiling MeOH and isolated. 3-*O*-Benzyl-14-methoxymorphinone (3) in the form of white-beige crystalls were obtained.

Mp: 180°-184°C

¹H-NMR (200 MHz, CDCl₃): δ [ppm] 7,41-7,26 (m, 5 arom. H)
6,71 (d, 1 arom. H, J= 8,3 Hz)
6,60 (d, 1 olef. H,J= 10,3 Hz)
6,56 (d, 1 arom. H, J= 8,3 Hz)
6,27 (d, 1 olef. H, J= 10,3 Hz)
5,149 (s, 2H, CH₂-Phenyl)
4,77(s,1H,H5)
3,28 (s, 3H, OCH₃)
2,47 (s, 3H, NCH₃)

### 14-O-Methyloxymorphone (4)

0.20 g (0.5 mmol) of 3-*O*-Benzyl-14-methoxymorphinone (**3**) were dissolved in a mixture of 20 mL of EtOH_{abs} and 0.23 mL of HCOOH at r.t. under argon. To the mixture obtained 0.16 g of Pd/C-catalyst (10%) were added and the mixture obtained was refluxed for 2.5 h. From the mixture obtained the catalyst was separated and from the solution obtained solvent was evaporated. The evaporation residue obtained was treated with 10 mL of H₂O and the mixture obtained was treated with 7 drops of conc. NH₃. The mixture obtained was extracted with CH₂Cl₂ (4x4 mL).The combined organic phases obtained were dried over Na₂SO₄ and solvent was evaporated. 14-*O*-Methyloxymorphone (**4**) was obtained in the form of a reddish solid.

Mp: 128°C

¹H-NMR (200 MHz, CDCl₃): δ [ppm] 6,71 (d, 1 arom. H, J= 8,0 Hz)
6,60 (d, 1 arom. H, J= 8,0 Hz)
5,30 (s, 1 H, OH)
4,66 (s, 1 H, H5)
3,35 (s, 3 H, OCH₃)
2,41 (s, 3 H, NCH₃)

14-O-Methyloxymorphone (**4**) in free form was converted into a hydrobromide salt by treatment in MeOH with 48% HBr. Mp and NMR of the hydrobromide obtained were identical with material obtained according to H. Schmidhammer et al. J. Med. Chem. 1984, 27, 1575-1579.

### Example 2

### 14-O-Methyloxymorphone from oripavine via 3-O-Benzyloripavine

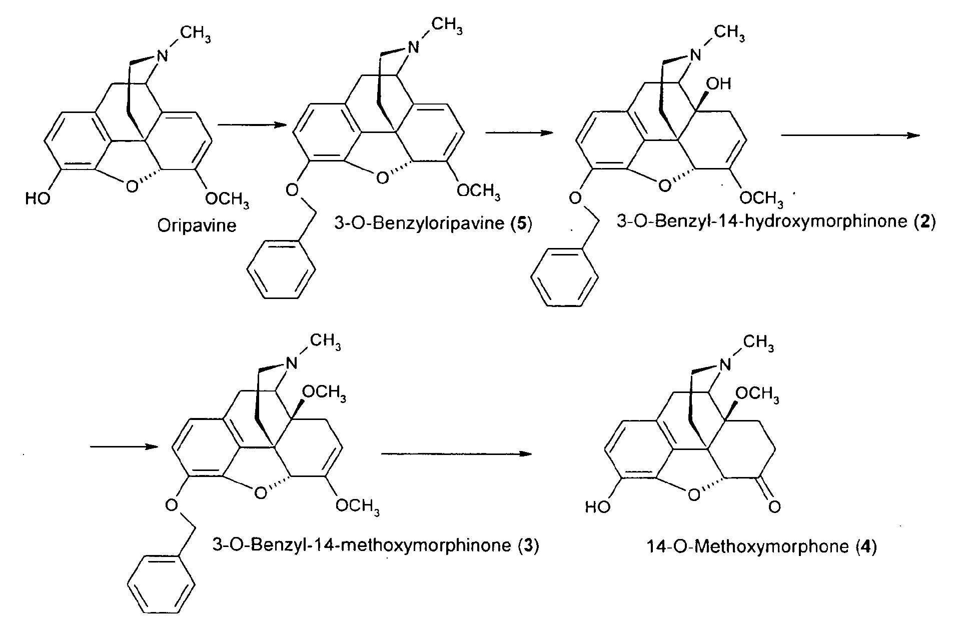

### 3-O-Benzyloripavine (5)

A solution of 0.5 g (1.7 mmol) of oripavine in a mixture of 16.7 mL of an aqueous 40% solution of 6.5 g of tetrabutylammoniumhydroxid and 10 mL of CH₂Cl₂ were treated under argon with 0.2 mL (0.3 g, 2.0 mmol) of benzyl chloride and the mixture obtained was stirred for 5 h at r.t.. The mixture obtained was poured onto 150 mL of H₂O and the mixture obtained was extracted with CH₂Cl₂ (4x10 mL). The combined organic phases obtained were washed with diluted NaOH (2x) and H₂O (3x, in total 200 mL), dried over Na₂SO₄ and from the mixture obtained solvent was evaporated. 0.57 g of a dark, oily residue were obtained and subjected to column chromatography (Silica gel 60, mobile phase CH₂Cl₂):MeOH:conc. NH₄OH = 95:5:0,25). 3-*O*-Benzyloripavine (**5**) was obtained in the form of a cream-colored foamy resin which was crystallized from MeOH.

Mp: 127°-128°C

¹H-NMR (CDCl₃): δ 7,46 - 7,26 (m, 5 aromat. H, Benzyl)
6,67 (d, 1 aromat. H, J= 8Hz)
6,53 (d, 1 aromat. H, J= 8Hz)
5,57 (d, 1 olefin. H, J= 6,4)
5,31 (s,H-C5)
5,19 (dd, 2 H, Phenyl-CH₂, J= 12,3 Hz)
5,05 (d, 1 olefin. H, J= 6,4)
3,62 (s, 3 H, OCH₃ - C6)
2,46 (s, 3 H, NCH₃)

### 3-O-Benzyl-14-hydroxymorphinone (2)

A solution of 1.40 g (3.6 mmol) of 3-*O*-benzyloripavine (**5**) in a mixture from 0.8 mL H₂O and 1.9 mL (2.28 g, 49.5 mmol) of HCOOH was treated dropwise with 0,43 mL (0.48 g) of a 35% H₂O₂ solution. The mixture obtained was stirred at r.t. for 2.5 h. The pH of the mixture obtained was adjusted to ca. pH = 9 by addition of NH₄OH under ice-cooling and the mixture obtained was stirred for 10 min in an ice bath. The mixture obtained was kept under cooling in a refrigerator for 17 h and a precipitate formed which was collected and dried. The precipitate obtained was dissolved in hot MeOH and the mixture obtained was treated with charcoal and filtered. From the filtrate obtained solvent was evaporated. Crystalline 3-*O*-benzyl-14-hydroxymorphinone (**2**) was obtained and recrystallized from MeOH to obtain colorless crystalline 3-*O*-benzyl-14-hydroxymorphinone (**2**) which was identical with material obtained in example 1 by melting point and ¹H-NMR.

14-*O*-Methyloxymorpone (**4**) was obtained from 3-*O*-benzyl-14-hydroxymorphinon (**2**) as described in example 1.

### Example 3

### 14-O-Butyloxymorphone

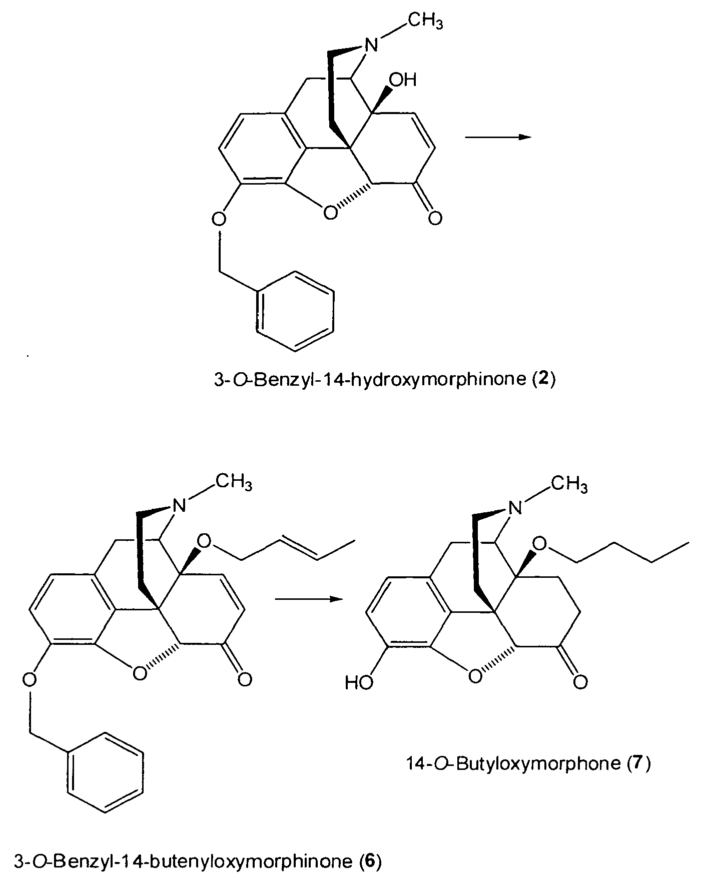

### 3-O-Benzyl-14-O-butenyloxymorphinone (6)

0.09 g (3.8 mmol) of NaH (obtained by several washings of 0.25 g of a 60% NaH-dispersion in oil with petrolether) were added at 0°C to a suspension from 7 mL DMFabs and 1.0 g (2.6 mmol) of -*O*-Benzyl-14-hydroxymorphonone (**2**). After 40 min 0.45 mL of a 85% crotyl bromide solution (0.5 g crotyl bromide, 3.8 mmol) were added dropise and the mixture obtained was stirred at 0°C for 1.5 h. To the mixture obtained 100 mL of a H₂O/ice mixture was carefully added and the mixture obtained was extracted with CH₂Cl₂ (5x10 mL). The organic phase obtained was washed with H₂O (4x25 mL), dried over Na₂SO₄ and from the mixture obtained solvent was evaporated. 3-*O*-Benzyl-14-*O*-butenyloxymorphinone (6) was obtained in the form of a yellow oil which was used without further purification in the next synthesis step.

¹H-NMR (CDCl₃): δ 7,37 - 7,26 (m, 5 aromat. H)
6,73 (d, 1 aromat. H, J= 5,2)
6,69 (d, 1 aromat. H, J= 5,2)
6,54(d, 1 olefin. H, J= 9,1)
6,19(d, 1 olefin. H, J= 9,1)
5,64- 5,53 (m, 2 H CH₂CHCHCH₃)
5,15 (s, 2 H, CH₂-Phenyl)
4,76 (s, H- C5)
2,46 (s, 3 H, NCH₃)
1,69 (d, 3 H, CH₂ CH₁ CH₁ CH₃ J= 5,6)

### 14-O-Butyloxymorphone (7)

6.0 mL (5.4 g, 67.2 mmol) of cyclohexa-1,4-diene were added dropwise under argon to a solution of 1.0 g (2.2 mmol) of 3-*O*-benzyl-14-*O*-butenyloxymorphinone (**6**) in 15 mL of EtOH_{abs} under stirring. To the mixture obtained 0.5 g of Pd/C-catalyst (10%) were added and the mixture obtained was stirred for 4 h at 100°C oil bath temperature. From the mixture obtained the catalyst was separated and from the filtrate obtained solvent was evaporated and the residue obtained was dried. 0.46 g of yellowish crystalls were obtained as a residue. Since from IR-spectra it was determined that the double bond was not fully hydrogenated the residue obtained was treated with 15 mL of EtOH_{abs}, the mixture obtained was treated with 4 mL of cyclohexa-1,4-diene and to the mixture 0.4 g of Pd /C-catalyst were added under argon. The mixture obtained was refluxed for 24 h at 100°C oil bath temperature. From the mixture obtained the catalyst was separated, from the filtrate obtained solvent was evaporated and the evaporation residue was dried under high vacuum. 0.33 g of a yellow-brownish oil were obtained as a residue. Since from IR-spectra from the residue obtained it was determined that the double bond was not totally hydrogenated, 10 mL of EtOH_{abs} were added to the residue obtained, 0.45 mL (0.6 g, 13.2 mmol) of HCOOH and 0,2 g of Pd charcoal were added under argon and the mixture obtained was refluxed for 7.5 h under argon. From the mixture obtained the catalyst was separated by filtration and the filtrate obtained was treated with 10 mL of H₂O. The pH of the mixture obtained was adjusted to an alkaline pH by addition of conc. ammonia and the mixture obtained was extracted with CH₂Cl₂ (4x8 mL). The combined organic phases obtained were dried over Na₂SO₄ and solvent was evaporated. The evaporation residue obtained was subjected to column chromatography (Silica gel 60. mobile phase CH₂Cl₂:MeOH:conc. NH₃ = 95:5:0,25). 14-*O-*Butyloxymorphone (**7**) was obtained in the form of a cream-colored foamy resin.

¹H-NMR (CDCl₃): δ 7,26 (s, CHCl₃ )
6,71 (d, 1 aromat. H, J= 8,4 Hz)
6,59 (d, 1 aromat. H, J= 8,4 Hz)
5,30 (s, 1 H, OH)
4,66(s,H-C5)
3,34 (t, CH₂ CH₂ CH₂ CH₃, J= 6,2 Hz)
2,37 (s, 3 H, NCH₃)
1,71 - 1,37 (m, 4 H, CH₂ CH₂CH₂CH₃)
0,96 (t, 3 H, CH₂ CH₂ CH₂ CH₃, J= 7 Hz)

### Example 4

### 14-O-Methyloxymorphone

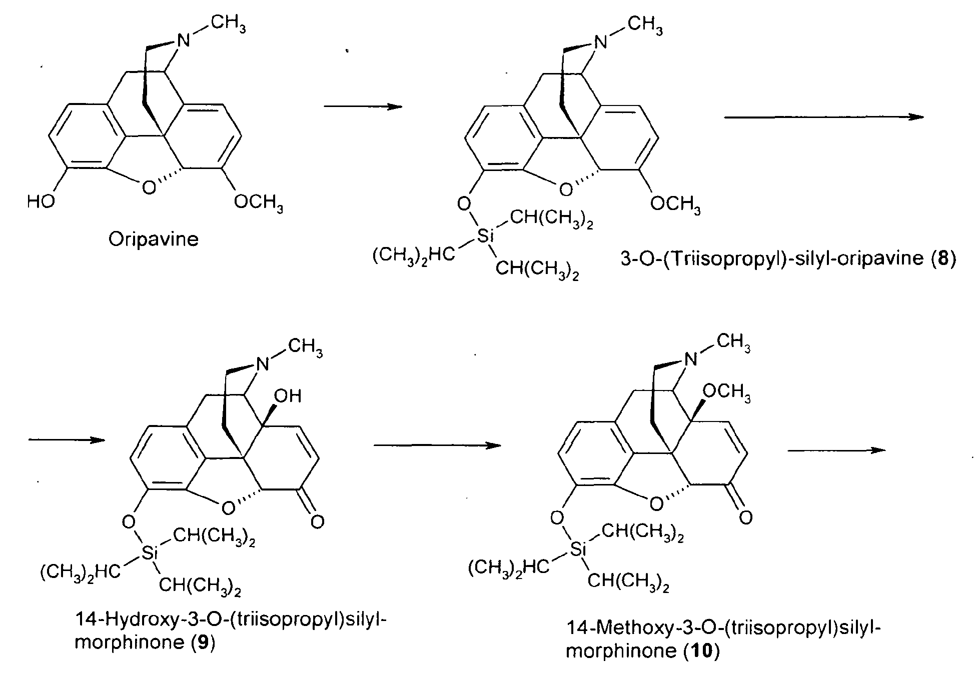

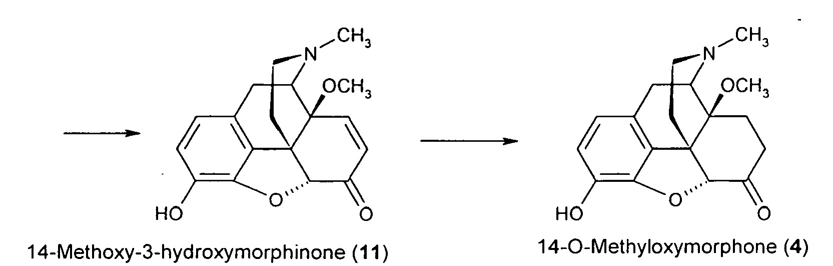

### 3-O-(Triisopropyl)silyloripavine (8)

To a solution from 8.0 g (10.2 mmol) oripavine in 70 mL of DMF_{abs}. 19 mL (107.6 mmol, 14.24 g) of diisopropylethylamine and 9.5 mL (40.4 mmol, 7.91 g) of chloro-triisopropylsilane were added dropwise under argon in an ice bath and the mixture obtained was stirred for 3.5 h at r.t.. The mixture obtained was poured onto 500 mL of H₂O and the mixture obtained was extracted with CH₂Cl₂ (4x25 mL). The combined organic phases obtained were washed with H₂O (4x 25 mL), dried over Na₂SO₄ and solvent was evaporated. The brown evaporation residue (19.60 g) obtained was kept in a refrigerator overnight and a precipitate was obtained. 3-*O*-(Triisopropyl)silyloripavine (**8**) was isolated in the form of colorless crystalls.

Mp: 94-96°C.

¹H-NMR (200 MHz, CDCI₃): δ [ppm] 6,61 (d. 1 arom. H, J= 8,3 Hz)
6,48 (d, 1 arom. H, J= 8,3 Hz)
5,52 (d, 1 olef. H, J= 6,4 Hz)
5.22 (s, 1 H, H5)
4,98 (d, 1 olef. H, J= 6,4 Hz)
3,55 (s, 3 H, OCH₃)
2,45 (s, 3 H. NCH₃)
1,07 (d, 18 aliph. H, Silyl 3x CH-(CH₃)₂, J= 6,2 Hz)

### 14-Hydroxy-3-O-(triisopropyl)silylmorphinone (9)

2.0 g of 3-*O*-(triisopropyl)silyloripavine (8) were dissolved in a mixture from 1.0 mL of H₂O and 2.2 mL of HCOOH under ice-cooling. To the mixture obtained 0.6 mL of 35% H₂O₂-solution was added dropwise and the mixture obtained was stirred for 10 h under ice-cooling. The mixture obtained was treated with concentrated NH₃, the pH was adjusted to 10-11 1 and the mixture obtained was kept overnight in a refrigerator. A cream-colored solid precipitated and was isolated. Crystalline 14-hydroxy-3-*O*-(triisopropyl)silylmorphinon (9) was obtained.

Mp: 162-168 °C.

¹H-NMR (200 MHz, CDCl₃): δ [ppm] 6,66 (d. 1 arom. H, J= 8,2 Hz)
6,60 (d, 1 olef. H, J= 10,1 Hz)
6,53 (d, 1 arom. H, J= 8,2 Hz)
6,15 (d, 1 olef. H, J= 10,1 Hz)
4,67 (s, 1 H, H5)
2,43 (s, 3 H, NCH₃)

1,05 (d, 18 aliph. H, Silyl 3x CH-(CH₃)₂, J= 6,4 Hz)

### 14-Methoxy-3-O-(triisopropyl)silylmorphinone (10)

0,3 g (0.7 mmol) of 14-hydroxy-3-*O*-(triisopropyl)silylmorphinone (9) were suspended under argon in 2 mL of DMF_{abs} and the mixture obtained was treated with 0.02 g (1.0 mmol) of NaH (obtained by seveal washings of 0.10 g of a 60% NaH-dispersion in oil with petrolether) in an ice bath and the mixture obtained was stirred for 40 min under ice-cooling. To the mixture obtained 1 mL (1.0 mmol, 0.12 g) of dimethylsulfate was added dropwise and the mixture obtained was stirred for 2 h. In order to remove starting material which was still present (determined by thin layer chromatography) the mixture obtained was treated with 0.02 g NaH (obtained by several washings of 0.10 g of a 60% NaH-dispersion in oil with petrolether) in an ice bath and the mixture obtained was stirred for 3 h under ice-cooling. To the mixture obtained small pieces of ice were added in order to stop the reaction and the mixture obtained was poured onto 50 mL of a H₂O/ice mixture. The mixture obtained was extracted with CH₂Cl₂ (5x6 mL), the combined organic phases were washed with H₂O (3x15 mL), dried over Na₂SO₄ and solvent was evaporated. The evaporation residue in the form of a brown oil obtained was subjected to column chromatography (Silica gel 60, mobile phase: CH₂Cl₂:MeOH:conc. NH₃ = 95:5:0,25). 14-Methoxy-3-*O-*(triisopropyl)silylmorphinone (10) in the form of an orange-red amorphous precipitate was obtained.

¹H-NMR (200 MHz, CDCI₃): δ 6,69 (d, 1 arom. H, J= 8,3 Hz) 6,61 (d, 1 arom. H, J= 8,3 Hz)

6,60 (d, 1 olef. H, J= 11,3 Hz)
6,28 (d, 1 olef. H, J= 11,3 Hz)
4,75 (s, 1H, H5)
3,27 (s, 3 H, OCH₃)
2,48 (s, 3 H, NCH₃)
1,26 (d, 18 H. Silyl 3x CH-(CH₃)2, J= 6,8 Hz)

### 14-Methoxy-3-hydroxymorohinone (11)

0.5 g (1.06 mmol) of 14-methoxy-3-*O*-(triisopropyl)silylmorphinone (**10**) were dissolved in 5 mL of MeOH, treated with 1.3 mL of conc. HCl and the mixture obtained was refluxed for 6 h in an oil bath of 85°C. MeOH was distilled off under reduced pressure and the remaining mixture was treated with 10 mL of H₂O. The pH of the mixture obtained was adjusted to an alkaline pH by addition of conc. ammonia solution and the mixture obtained was extracted with 80 mL of CH₂Cl₂ several times. The organic phase obtained was washed with H₂O and saturated NaCl solution, dried over Na₂SO₄ and solvent was evaporated. 14-Methoxy-3-O-hydroxy-morphinone (11) was obtained in the form of a brown, amorphous solid.

¹H-NMR (CDCl₃, 200 MHz): δ 6.69 (d, 1 aromat. H, J=8.0 Hz)
6.63 (d, 1 olefin. H, J=10.2 Hz)
6.59 (d, 1 aromat. H, J=8.0 Hz)
6.28 (d, 1 olefin. H, J=10.2 Hz)
4.77 (s, 1 H, C5)
3.27 (s, 3 H, C14-OCH₃)
2.48 (s, 3 H, NCH₃)

### 14-O-Methyloxymorphone (4)

257 mg (0.82 mmol) of 14-methoxy-3-O-hydroxy-morphinone (**11**) were dissolved in MeOH and the mixture obtained was treated with hydrogen in the presence of 30 mg of 10% Pd/C-catalyst for 2.5 h at 30 psi. From the mixture obtained the catalyst was filtrated off and from the filtrate obtained solvent was evaporated. 223 mg of brownish crystalls were obtained which were dissolved and subjected to chromatography (Silica gel 60, CH₂Cl₂:MeOH:conc. NH₃ = 95:5:0.25). 14-O-Methyloxymorphone (4) was obtained in the form of reddish crystalls.

¹H-NMR (CDCl_{3.} 200 MHz): δ 6.72 (d, 1 aromat. H, J=8.3 Hz)
6.61 (d, 1 aromat. H, J=8.3 Hz)
5.31 (s, 1 H, OH)
4.66 (s, 1 H, C5)
3.35 (s, 3 H, C14-OCH₃)
2.42 (s, 3 H, NCH₃)

### 14-O-Methyloxymorphone (4) in the form of a hydrobromide salt

50 mg (0.16 mmol) of 14-O-methyloxymorphone (**4**) were dissolved in 10 drops of MeOH under warming and the solution obtained was treated with 20 µl of 48% HBr. The mixture obtained was kept for 48 h at 4°C. A precipitate formed and was isolated and dried. 14-O-Methyloxymorphone (**4**) in the form of a hydrobromide in crystalline form (white-brownish color) was obtained. Mp: > 300°C (decomposition)

### Example 5

### 14-O-Methyloxymorphone

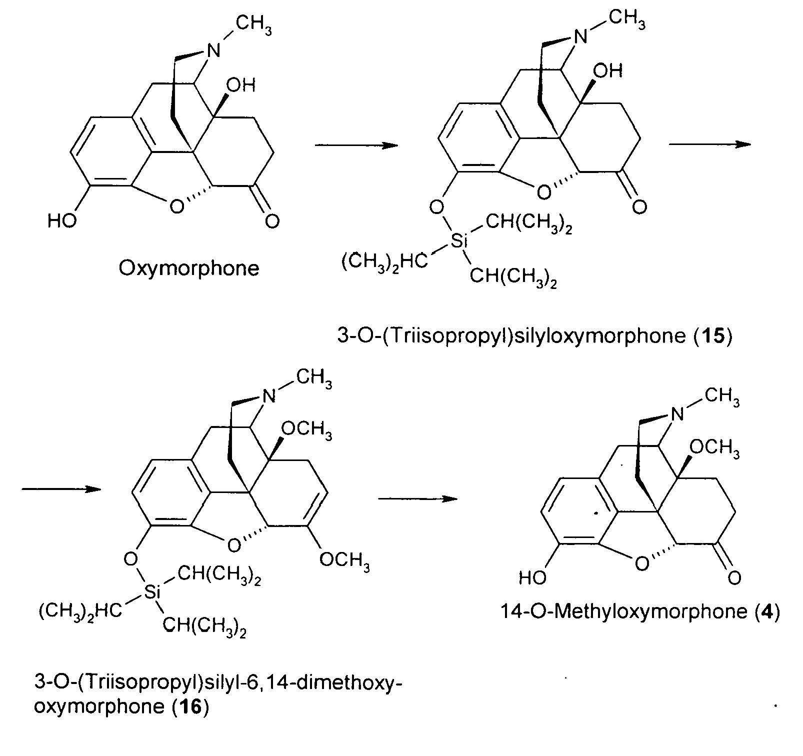

### 3-O-(Triisopropyl)silyloxymorphone (15)

9.5 mL (53.1 mmol, 7.02 g) of diisopropylethylamine and 4.5 mL (19.9 mmol, 3.90 g) of chloro-triisopropylsilan were added to a solution of 4.0 g (13.3 mmol) of oxymorphone in 20 mL of DMF_{abs} under ice-cooling and the mixture obtained was stirred at r.t. for 3.5 h. The mixture obtained was treated with 100 mL of H₂O and the mixture obtained was extracted with CH₂Cl₂ (3x40 mL). The organic phases obtained were washed with H₂O (6x50 mL), dried over Na₂SO₄ and solvent was evaporated. 3-*O*-(Triisopropyl)silyloxymorphone (**15**) was obtained in the form of a brown viscous oil.

¹H-NMR (200 MHz, CDCI₃): δ [ppm] 6,66 (d, 1 arom. H, J= 7,7 Hz)
6,54 (d, 1 arom. H, J= 7,7 Hz)
4,58 (s, 1 H, H5)
2,40 (s, 3 H, NCH3)
1,09 (d, 18 aliph. H, Silyl 3x CH-(CH₃)₂J= 9.8 Hz)

### 6.7-Didehydro-6,14-dimethoxy-4,5α-epoxy-N-methyl-3-O-(triisopropyl)silylmorphinan (16)

3.0 g (6.6 mmol) of 3-*O*-(triisopropyl)silyloxymorphone (**15**) were suspended under argon in 15 mL of DMF_{abs} and the mixture obtained was treated with 0.48 g (19.8 mmol) of NaH (obtained by several washings of 1.0 g of a 60% NaH-dispersion in oil with petrolether) in an ice bath and the mixture obtained was stirred for 40 min under ice cooling. The mixture obtained was treated with 1.9 mL (19.8 mmol, 2.47 g) of dimethylsulfate and the mixture obtained was stirred for 1.5 h under ice cooling. The mixture obtained was carefully treated with 80 mL of H₂O and the mixture obtained was extracted with CH₂Cl₂ (4x 13 mL). The combined organic phases obtained were washed with H₂O (3x80 mL), dried over Na₂SO₄ and solvent was evaporated. 6,7-Didehydro-6,14-dimethoxy-4,5α-epoxy-N-methyl-3-*O-*(triisopropyl)silylmorphinan (16) in the form of a brown, viscous oil was obtained.

### 14-O-Methyloxymorphone (4)

6,7-Didehydro-6,14-dimethoxy-4,5α-epoxy-N-methyl-3-*O*-(triisopropyl)silylmorphinan (**16**) in the form of a brown, viscous oil was dissolved in 10 mL of MeOH and the mixture obtained was treated with 2.6 mL (84.9 mmol, 3.10 g) of conc. HCl. The mixture obtained was refluxed for 6 h at 100°C (bath temperature). From the mixture obtained solvent was evaporated, the dark brown evaporation residue was treated with 10 mL of H₂O and the pH of the mixture was adjusted to an alkaline pH by addition of conc. NH₃. The mixture obtained was extracted with CH₂Cl₂ (5x5 mL) and the combined organic phases obtained were extraced with diluted NaOH (3x20 mL). The combined aqueous phases obtained were treated with conc. HCl and an acidic pH was adjusted. The mixture obtained was treated with conc. NH₃ and an alkaline pH was adjusted. The mixture obtained was extracted with CH₂Cl₂ (4x8 mL), the combined organic phases obtained were dried over Na₂SO₄ and from the mixture obtained solvent was evaporated. 14-*O*-Methyloxymorphone (4) was obtained in the form of an amorphous powder.

¹H-NMR (200 MHz, CDCl₃): δ [ppm] 6,71 (d, 1 arom. H, J= 8,0 Hz)
6,60 (d, 1 arom. H, J= 8,0 Hz)
5,30 (s, 1 H, OH)
4,66 (s, 1 H, H5)
3,35 (s, 3 H, OCH₃)
2,41 (s, 3 H, NCH₃)

14-*O*-Methyloxymorphone (4) was dissolved in MeOH and converted into the hydrobromide by addition of 48% HBr. Mp and NMR were identical with authentical material (H. Schmidhammer et al. J. Med. Chem. 1984, 27, 1575-1579).

### Example 6

### 14-O-Benzyloxymorphone

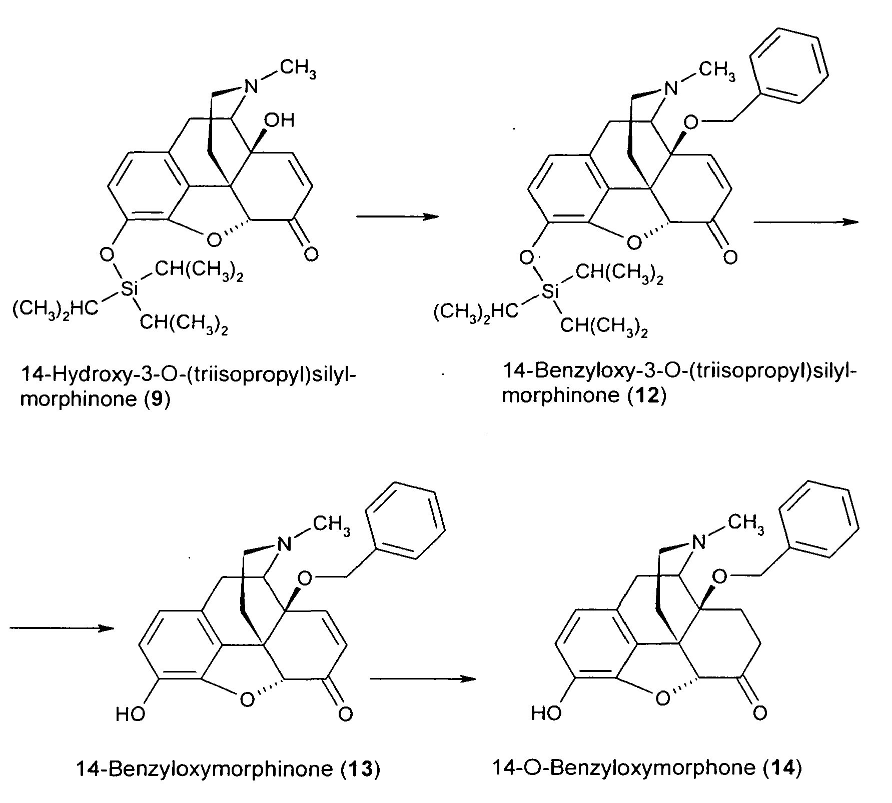

### 14-Benzyloxy-3-O-(triisopropyl)silylmorphinone (12)

A solution of 500 mg of 14-hydroxy-3-O-(triisopropyl)silyl-morphinone (**9**) (1.1 mmol) in 20 mL of anhydrous DMF was stirred under argon and cooled to 0°C (ice bath temperature) for 20 min. To the mixture obtained 130 mg (5.4 mmol) of NaH (obtained from 217 mg of 60% dispersion in oil by 3 washings with petrolether) were added and the mixture obtained was stirred for 15 min at 0°C and further 15 mi at r.t.. The ice bath was replaced by an ice/brine bath and the mixture obtained was cooled for 10 min to -8°C. To the mixture obtained 0.16 mL (1.4 mmol) of benzylbromide were added and the mixture obtained was stirred for 3.5 h. In the mixture obtained the excess of NaH was destroyed by addition of ice and H₂O (25 mL), the mixture obtained was extracted with CH₂Cl₂ (4x25 mL) and the combined organic layers were washed with H₂O (5x150 mL), 1x100 mL brine and dried (Na₂SO₄). From the mixture obtained solution was evaporated under reduced pressure. 14-Benzyloxy-3-O-(triisopropyl)silylmorphinone (12) in the form of a yellow oil was obtained.

¹H-NMR (CDCI₃): δ (ppm) 7.38 - 7.24 (m, 5H arom., benzyl), 6.79 (d, J=10.1 Hz, 1H, H-C8), 6.65 (d, J=8.0 Hz, 1H, *H*-C1), 6.53 (d, J=8.0 Hz, 1H, *H*-C2), 6.13 (d, J=10. 1 Hz, 1H, H-C7), 5.15 (s, 1H, *H*-C5), 4.68 (d, J=11.0Hz, 1H, C(14)-O-C*H*₂-Ph). 4.45 (d, J=11.0Hz. 1H. C(14)-O-C*H₂*-Ph), 2.46 (s, 3H, N-C*H*₃), 1.04 (s, 18H, 6xC*H*₃, triisopropylsilyl)

### 14-Benzyloxymorphinone (13)

A solution of 185 mg (0.34 mmol) of 14-benzyloxy-3-O-(triisopropyl)silyl-morphinone (**12**) in 6 mL of MeOH (absolute for synthesis) and 2 mL concentrated HCl were refluxed for 10 h. To the mixture obtained 10 mL of H₂O were added and the mixture obtained was adjusted to an alkaline pH by addition of NH₄OH. The mixture obtained was extracted with CH₂Cl₂ (3x20 mL) and the combined organic layers were washed with H₂O (2x100 mL) and brine (1x100 mL) and dried (Na₂SO₄) From the mixture obtained solvent was evaporated under reduced pressure to give a dark yellow oil (140 mg) which was subjected to column chromatography (Silica gel 60 (Mesh 230 - 400, grading 0.04 - 0.063), mobile phase: CH₂Cl₂ / H₂O / conc. NH₃. = 95 : 5 : 0.25) for purification. Adequate fractions were collected and solvent was evaporated under reduced pressure. 14-Benzyloxy-morphinone (**13**) was obtained in the form of a yellow oil.

¹H-NMR (CDC₃): δ (ppm) 7.39-7.26 (m, arom, 5H, benzyl), 6.83 (d, J=10.3 Hz, 1H, H-C(8)), 6.68 (d, J=8.2 Hz, 1H, *H*-C(1)), 6.57 (d, J=8.2 Hz, 1H, *H*-C(2)), 6.18 (d, J=10.3 Hz. 1H, *H*-C(7)), 4.78 (s, 1H, *H*-C(5)), 4.68 (d, J=11Hz, C(14)-O-CH₂-Ph), 4.47 (d, J=11Hz, C(14)-O-C*H*₂-Ph), 2.47 (s, 3H, N-C*H*₃)

### 14-Benzyloxymorphone (14)

A mixture of 40 mg of 14-benzyloxy-morphinone (**13**), 10 mg of Pd/C catalyst (10%) and 20 mL of MeOH was hydrogenated at 30 psi (r.t.) for 20 h. From the mixture obtained the catalyst was filtrated off and from the filtrate obtained solvent was evaporated under reduced pressure. 14-Benzyloxy-morphone (**14**) was obtained in the form of an orange-brown foam. (R. Lattanzi et al., J. Med. Chem., Vol. 48, 3372-3378, 1984)

¹H-NMR (DMSO-d₆): δ (ppm) 7.51-7.28 (m, arom, 5H, benzyl), 6.70 (d, J=8.3 Hz, 1H, H-C(1)), 6.59 (d, J=8.3 Hz, 1H, *H*-C(2)), 4.82 (s, 1H, H-C(5)), 4.67 (d, J=11Hz, 1H, C(14)-O-CH₂-Ph), 4.48 (d, 1H, J= 11Hz, 1H, C(14)-O-C*H*-Ph), 2.47 (s, 3H, N-C*H*₃).

## Claims

1. A process for the production of an 14-oxygenated morphinan-6-one, wherein a methylether group in position 3 of the phenyl ring which has to be cleaved to obtain the hydroxy group, and wherein the protection of the hydroxy group in position 3 of the phenyl ring in the presence of a ketal group is avoided.

2. A process according to claim 1, wherein an 14-oxygenated morphinan-6-one is a compound of formula 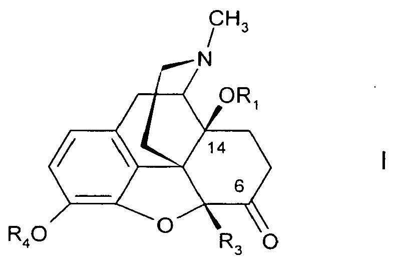 wherein
R₁ is alkyl, alkenyl, alkynyl, cycloalkylalkyl, aralkyl, alkanoyl, alkenoyl, alkynoyl, cycloalkylcarbonyl, aroyl, or alkynoyl substituted by cycloalkyl,
R₃ is alkyl, alkenyl, alkynyl, cycloalkylalkyl or aralkyl, or alkynoyl substituted by cycloalkyl, and
R₄ is hydrogen or has the meaning of R₁

3. A process according to claim 2, wherein in a compound of formula I R₁ is alkyl, or aralkyl.

4. A process according to any one of claims 2 or 3, wherein in a compound of formula I R₃ is hydrogen.

5. A process according to any one of claims 2 to 4, wherein in a compound of formula I R₄ is hydrogen.

6. A process for the production of a compound of formula I as claimed in any one of claims 2 to 5. comprising
A1) deprotecting the protected hydroxy group in position 3 of the phenyl ring in a compound of formula 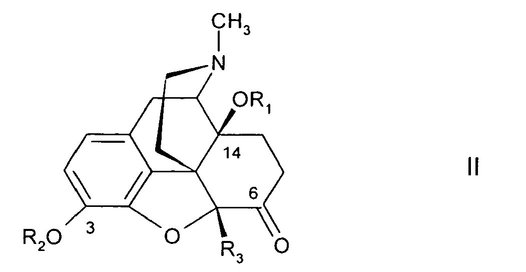 wherein R₁ and R₃ are as defined in any one of claims 2 or 3 and R₂ is a hydroyxy protecting group, or
A2) deprotecting the protected hydroxy group in position 3 of the phenyl ring and hydrolizing the enol ether in position 6 in a compound of formula 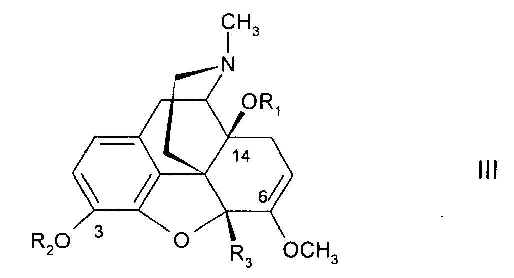 wherein R₁ and R₃ are as defined above,
and isolating a compound of formula I wherein R₁ is as defined above and is R₄ hydrogen from the reaction mixture, and, optionally
B) etherifying or esterifying the hydroxy group in position 3 of the phenyl ring in a compound of formula III to form a group OR₄, wherein R₄ has the meaning of R₁ as defined above.

7. A process according to claim 6, wherein
- in step A1) a compound of formula II is obtained from a compound of formula 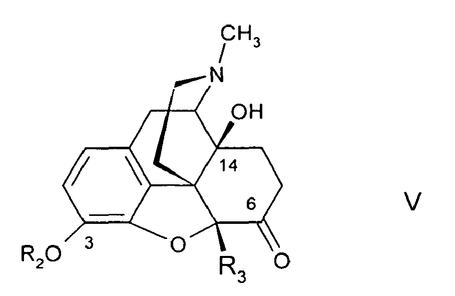 wherein R₂ and R₃ are as defined in claim 4;
- in step A2) a compound of formula III is obtained from a compound of formula V.

8. A process according to claim 6 wherein a compound of formula I is a compound of formula 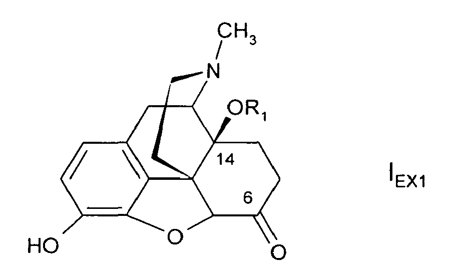 wherein R₁ is as defined in any one of claims 1 to 5, comprising subjecting a compound of formula 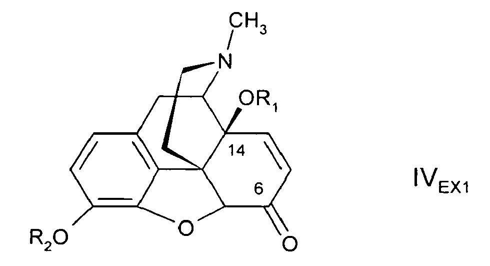 wherein R₁ is as defined above and R₂ is a hydroxy protecting group, to catalytic transfer hydrogenation of the double bond and splitting off the protection group from the hydroxy group attached to the phenyl ring,
wherein optionally a compound of formula IV_{Ex1} is obtained from a compound of formula 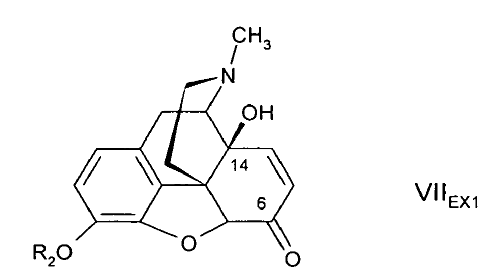 wherein R₂ is as defined above by reaction with a compound of formula L-R₁, wherein L is a leaving group;
wherein optionally a compound of formula VII_{EX1} is obtained from 14-hydroxymorphinone (**1**) by protection of the hydroxy group in position 3 of the phenyl ring.

9. A process according to claim 6 wherein a compound of formula I is a compound of I_{EXI}
as defined in claim 8, and is obtained from a compound of formula 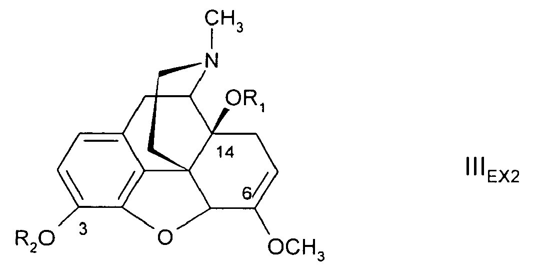 wherein R₁ and R₂ are as defined in claim 8, by acidic hydrolysis;
wherein a compound of formula III_{EX2} optionally is obtained from a compound of formula 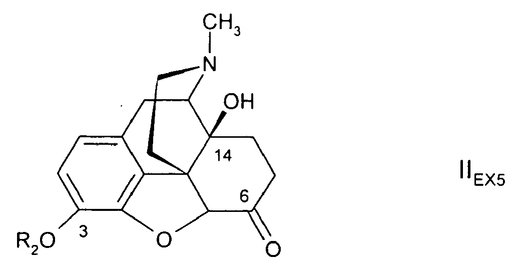 wherein R₂ is as defined above by reaction of the hydroxy group in position 14 with a compound of formula R₁-L, wherein R₁ is as defined above and L is a leaving group;
wherein a compound of formula II_{Ex5} optionally is obtained from oxymorphine by protection of the hydroxy group in position 3 of the phenyl ring.
